Europäisches Patentamt

European Patent Office

Office européen des brevets

(19)

(11)  **EP 0 804 287 B1**

(12)  **EUROPÄISCHE PATENTSCHRIFT**

(45) Veröffentlichungstag und Bekanntmachung des
Hinweises auf die Patenterteilung:
**22.07.1998 Patentblatt 1998/30**

(21) Anmeldenummer: **95942035.7**

(22) Anmeldetag: **15.12.1995**

(51) Int. Cl.⁶: $B01J\ 27/138$, $C07C\ 5/48$, $C07C\ 11/04$

(86) Internationale Anmeldenummer:
**PCT/DE95/01840**

(87) Internationale Veröffentlichungsnummer:
**WO 96/22161 (25.07.1996 Gazette 1996/34)**

(54) **KATALYSATOR FÜR DIE OXIDATIVE DEHYDRIERUNG VON PARAFFINISCHEN KOHLENWASSERSTOFFEN UND VERWENDUNG DIESES KATALYSATORS**

CATALYST FOR OXIDATIVE DEHYDROGENATION OF PARAFFINIC HYDROCARBONS AND USE OF THIS CATALYST

CATALYSEUR POUR LA DESHYDROGENATION OXYDANTE D'HYDROCARBURES PARAFFINIQUES ET UTILISATION DE CE CATALYSEUR

(84) Benannte Vertragsstaaten:
**DE DK IT NL**

(30) Priorität: **18.01.1995 DE 19502747**

(43) Veröffentlichungstag der Anmeldung:
**05.11.1997 Patentblatt 1997/45**

(73) Patentinhaber:
 • **MANNESMANN Aktiengesellschaft**
   **40213 Düsseldorf (DE)**
 • **K.T.I. Group B.V.**
   **2715 AB Zoetermeer (NL)**

(72) Erfinder:
 • **HERSKOWITZ, Mordechay**
   **85025 Maitar (IL)**
 • **LANDAU, Miron**
   **84110 Beer-Sheva (IL)**

 • **KALIYA, Mark**
   **84110 Beer-Sheva (IL)**

(74) Vertreter:
  **Meissner, Peter E., Dipl.-Ing. et al**
  **Meissner & Meissner,**
  **Patentanwaltsbüro,**
  **Hohenzollerndamm 89**
  **14199 Berlin (DE)**

(56) Entgegenhaltungen:
  **EP-A- 0 205 765          EP-A- 0 206 042**
  **US-A- 4 777 319**

 • **APPLIED CATALYST, Bd. 79, 1991 AMSTERDAM, Seiten L1-L5, XP 000567613 CONWAY ET AL. 'Selecrive oxidation of methane and ethane over Li+ - MgO - Cl- catalysts promoted with metal oxides' in der Anmeldung erwähnt**

**Beschreibung**

**Gebiet der Erfindung**

Die Erfindung bezieht sich auf ein Verfahren zum oxidativen Dehydrieren (und Cracken als Nebenreaktion) von $C_2$ - $C_5$ paraffinischen Kohlenwasserstoffen (reine Kohlenwasserstoffe oder Mischungen wie LPG-Flüssiggas) zu $C_2$ - $C_5$ Olefinen, wobei ein neuartiger Katalysator penutzt wird, der sich durch hohe Paraffin-Konversion und hohe Olefin-Selektivität auszeichnet.

**Hintergrund der Erfindung**

Die US 4 524 236 offenbart ein Verfahren zur oxidativen Dehydrierung (Oxydehydrierung) von Ethan, in welchem als Katalysator eine kalzinierte Zusammensetzung der Elemente Mo, V, Nb, Sb und X = Li, Sc, Na, Be, Mg, Ca, Sr, Ba, Ti, Zr, Hf, Y, Ta, Cr, Fe, Co, Ni, Ce, Zn, Cd, Hg, Al, Tl, Pb, As, Bi, U, W, Te. Dieser Katalysator liefert eine Konversion von bis zu 73 % bei einer Selektivität von 69 % für Ethylen bei 400 °C und einer gewichtsbezogenen Raumgeschwindigkeit (WHSV) von 1,5 h$^{-1}$. In diesem Patent wird darauf hingewiesen, daß der Katalysator im wesentlichen beschränkt ist auf die Oxydehydrierung von Ethan zu Ethylen, weil er Propan, n-Butan und i-Butan nicht effizient oxydehydriert, sondern diese Gas zu Kohlendioxyd und anderen oxidierten Kohlenstoffprodukten verbrennt.

Die Veröffentlichung "Selective oxidation of methane and ethane of Li$^+$-MgO-Cl$^-$catalysis promoted with metal oxides" von S.J. Conway, D.J. Wang und J.H. Lunsford, in Applied Catalysis A79, S. L1 -L5, 1991. offenbart Katalysatoren zur Oxydehydrierung von Ethan zu Ethylen, die bestehen aus Magnesiumoxid und Lithiumoxid, Chlor und anderen Metallen aus der Gruppe La, Nd, und Dy. Dieser Katalysator liefert eine Konversionsrate von 83,8 % für Ethan mit einer Selektivität für Ethylen von 63,8 % bei einer Temperatur von 585 °C und einer WHSV von 0,18 h$^{-1}$ Über die Leistung dieses Katalysators für die Oxydehydrierung von LPG-Flüssiggaskomponenten gibt es keine näheren Informationen.

Die US 4 777 319 offenbart einen Katalysator auf Vanadium-Basis für die Oxydehydrierung von $C_2$ - $C_8$-Paraffinen bei Temperaturen von 300 bis 700°C. Der Katalysator hat die Formel $M_3 (VO_4)_2$ und/oder $MV_2O_6$, wobei M eines der Elemente Mg, Zn, Ca, Pb oder Cd ist. Die Erfinder dieses Katalysators offenbaren detailliertere Informationen in später erschienen Veröffentlichungen:

"Selective Oxidative Dehydrogenation of Butane over V-MG-O Catalysts" von M.A. Chaar, D. Partel, M.C. Kung und H.H. Kung, (Journal of Catalysis 105, S. 483-498, 1987), "Selective Oxidative Dehydrogenation of Propane over V-MG-O Catalysts" von M.A. Chaar, D. Partel und H.H. Kung, (Journal of Catalysis 109, S. 463-467, 1988) und "Selectivity Patterns in Alkane Oxidation over $Mg_3(VO_4)_2$ - MgO, $Mg_2V_2O_7$ and $(VO)_2P_2O_7$" von P.M. Michalakos, M.C. Kung, I. Jahan und H.H. Kung, (Journal of Catalysis 140, S. 226-242, 1993). Diese Aufsätze offenbaren Mischoxidkatalysatoren, die Vanadium und Magnesium enthalten. Die Katalysatoren sind aktiv bei Temperaturen im Bereich von 475 bis 540 °C für die Oxydehydrierung von Propan, Butan und Isobutan, wobei sich bei 540 °C und WHSV = 2h$^{-1}$ eine Konversion von Butan in Höhe von bis zu 58,9 %, bei Propan eine Konversion von 35,8 % und bei 500 °C und WHSV = 6,5 h$^{-1}$ für Isobutan eine Konversion von 12 % mit einer Selektivität für korrespondierende Olefine und Butadien von 48,8 % bzw. 42,4 % bzw. 53,0 % ergibt. Es gibt keine Informationen über die Konversion von LPG-Flüssiggas auf V-MgO-Katalysatoren. Auf der Basis von Daten für die reinen Komponenten kann geschlossen werden, daß bei LPG-Flüssiggas, das ungefähr 50 % Propan enthält, eine Konversion von 40 % und eine Selektivität von 50 % erreicht werden können bei einem Olefin-Ertrag von ungefähr 20 % je Durchlauf bei WHSV = 2h$^{-1}$. Dies wurde durch Tests bestätigt, die im Rahmen der Untersuchungen für die vorliegende Erfindung durchgeführt wurden.

Ein wichtiges Merkmal dieser V-Mg-O-Katalysatoren ist die hohe Selektivität des Oxydehydrierungspfades. Es wurden keine Crack-Reaktionen beobachtet. Ein weiteres Merkmal dieser Katalysatoren ist, daß das Hauptprodukt der Dehydrierung von n-Butan Butadien ist, wobei die Selektivität für $C_4H_6$ 37,7 % bei einer Gesamtselektivität von 48,8 % und die Ausbeute von Butadien unter allen Dehydrierungsprodukten 77,2 % betragen.

Die Veroffentlichung von D. Bhattacharyya, S.K. Bej und M.S. Rao "Oxidative dehydrogenation of n-butane to butadiene. Effect of different promoters on the performance of vanadium - magnesium oxide catalysts", (Applied Catalysis A87, S. 29-43, 1992) offenbart einen Mischoxidkatalysator, der eine Mischung aus Vanadium, Magnesium und einer dritten Komponente umfaßt, die gebildet wird aus Mo, Cr und Ti oder aus Cr und Ti. Dieser Katalysator liefert bei 570 °C und WHSV = 0,8 h$^{-1}$ eine Konversion von 59 % und eine Selektivität von 53 % bei einer Ausbeute an Odydehydrierungsprodukten von 33,8 %, von denen 70 % Butadien ist.

Die Untersuchungen zur vorliegenden Erfindung haben gezeigt, daß im Gegensatz zu dem aus der US 4 524 236 bekannten Katalysator der Li-Mg-X-Cl-Katalysator, der aus Applied Catalysis A79, S. L1 - L5, 1991 bekannt ist und für die Oxydehydrierung von Ethan entwickelt wurde, bei der LPG-Konversion eine hohe Selektivität für Olefine bei 600 °C und WHSV = 0,18 h$^{-1}$ aufweist. Die Erhöhung der WHSV für das LPG verursacht einen drastischen Abfall der LPG-Konversion: Bei WHSV = 1 h$^{-1}$ und 600 °C betrug die Konversion 10,9 % bei etwa derselben Selektivität von 80 %.

Weitere Merkmale dieses Li-Mg-X-Cl-Katalysators sind das partielle Cracken von Butan, Propan und Isobutan unter den Bedingungen der Oxydehydrierung, wobei korrespondierende niedrige Olefine und Methan erhalten werden und vernachlässigbare Mengen an Butadien in den Oxydehydrierungsprodukten.

Zusammenfassend ist festzustellen, daß gemäß den verfügbaren Daten der beste Katalysator es erlaubt, eine hohe LPG-Konversion bei niedriger Selektivität für Olefine bei einer WHSV von ungefähr 2 h$^{-1}$ (V-Mg-O-Basis-Katalysator) oder eine hohe Selektivität für Olefine bei niedriger Konversion bei einer WHSV > 1 h$^{-1}$ (Li-Mg-X-Cl-Basis-Katalysator) zu erreichen.

## Aufgabe der Erfindung

Es ist Aufgabe der Erfindung, einen neuartigen Katalysator und die Verwendung dieses Katalysators in einem Verfahren für das oxidative Dehydrieren (und Cracken) von $C_2$-$C_5$-Paraffinen zu $C_2$-$C_5$-Olefinen bei hoher Konversionsrate und gleichzeitig hoher Selektivität für Olefine anzugeben.

## Zusammenfassung der Erfindung

Die vorliegende Erfindung stellt ein Verfahren und einen oxidischen Katalysator für die oxidative Dehydrierung und das Cracken von C2-C5-Paraffinen (artreine Kohlenwasserstoffe oder Mischungen wie LPG-Flüssiggas) zu C2-C5-Olefinen in einer Gasphase zur Verfügung. Der Katalysator ist kalziniert und hat eine Zusammensetzung gemäß der Formel $X_a\, X_b\, Z_c\, A_d\, O_x$, wobei gilt:

| | |
|---|---|
| X | = Zirkonium und/oder Hafnium |
| Y | = Element der Gruppe der Lanthaniden und/oder der Gruppen IVa der Va (Ce, La, Nd, Dy, Sn, Pr, Sb, Pb, ) |
| Z | = Element der Gruppe I (Li, Na, K....) |
| A | = Element der Gruppe VII (Cl, Br, J....) |

und

| | |
|---|---|
| a | = 0,4 - 0,9 |
| b | = 0,005 - 0,3 |
| c | = 0,05 - 1,5 |
| d | = 0,05 - 0,8 und |
| x | = eine Zahl, die durch die Valenz-Erfordernisse der Metalle und Halogene bestimmt ist. |

Die Werte von a, b, c und d bestimmen die relativen Mol-Anteile der Elemente X, Y, Z und A im Katalysator. Die Elemente außer den Halogenen kommen dabei in Verbindung mit Sauerstoff in Form von verschiedenen Oxiden oder als Halogenide vor.

Die Erfindung beinhaltet auch die Herstellungsweisen für den Katalysator in Form gepreßter, etwa kugelförmiger Pellets, von Extrudaten mit einer Bindemittelzugabe oder auf einem Träger, wobei dieser aus einer der oxidischen Komponenten des katalytischen Materials allein oder mit Bindemittelzugabe oder aber aus einem separaten Trägermaterial besteht. Die Erfindung ist gekennzeichnet durch den Katalysator und umfaßt die Betriebsbedingungen Temperatur T, Druck P, gewichtsbezogene Raumgeschwindigkeit WHSV, das Mol-Verhältnis von Sauerstoff zu Kohlenwasserstoffen, die Zugabe eines Inertgases oder Wassers und spezielle Maßnahmen zur Aufrechterhaltung der Katalysatorstabilität und die Zuführung zum Festbett- oder Fließbett-Reaktor.

## Beschreibung der Erfindung

Die Wahl der Komponenten, die benutzt werden, kann ebenso einen signifikanten Einfluß auf die Leistung eines Katalysators haben, wie das spezielle Verfahren, welches bei der Herstellung und Aktivierung des Katalysators angewendet wurde. Die Elemente der Katalysatorzusammensetzung liegen in Verbindung mit Sauerstoff oder Halogenen als Oxide oder Halogenide vor. Vorzugsweise wird der Katalysator hergestellt aus einer Lösung der löslichen Komponenten, die die Z- und A-Komponenten umfassen, sowie den unlöslichen pulverförmigen Oxiden der X- und Y-Komponenten. Die Y-Komponenten können auch als lösliche Verbindungen eingeführt werden. Die Lösung ist vorzugsweise ein wäßriges System bei pH 1 - 12 und mehr, vorzugsweise bei pH 3 - 6. Die Temperatur kann 20 bis 100 °C dabei betragen. Im Regelfall wird eine Mischung der Verbindungen, die die Elemente beinhalten, vorbereitet durch Lösung einer ausreichend großen Menge der löslichen Verbindungen und durch Dispergieren der unlöslichen Verbindungen, um das gewünschte Mol-Verhältnis der Elemente im Katalysator herzustellen. Der Katalysator wird dann erhalten durch Entfernung des Wassers oder eines anderen Lösungsmittels aus der Lösung bei einer Temperatur ungefähr im Bereich von 70 bis 100 °C. Der nasse Katalysator wird bei 110 bis 150 °C, vorzugsweise bei 130 °C, an Luft oder in Sauerstoff wahrend einer Zeit von 10 bis 24 Stunden getrocknet. Eine Erhöhung der Temperatur verkürzt die Synthesezeit. Der trockene Feststoff wird gemahlen zu einem Pulver von 400 bis 800 µm Korngröße. Die Kalzinierung wird vorzugsweise in mehreren Schritten ausgeführt. Im ersten Schritt wird die Kalzinierung an Luft oder Sauerstoff bei Temperaturen von 250 bis 600 °C. vorzugsweise bei etwa 400 °C, für eine Zeit von 0,5 bis 5 Stunden, vorzugsweise 2 Stunden ausgeführt. Dem folgt eine zusatzliche Kalzinierung bei einer Temperatur von 620 - 850°C, vorzugsweise 750 °C, über eine Zeit von 10 - 24 Stunden.

Das Material wird gepreßt und zu Kügelchen eingeformt unter Zugabe von Feuchtigkeit, oberflächenaktiven Stoffen und Plastifzierern oder geformt durch Extrusion unter Zugabe eines Bindemittels. Zu der Gruppe der verwendbaren Bindemittel für den Katalysator gehören die Oxide von Silizium, Aluminium, Zirkonium, Titan, Hafnium und Mischungen davon. In einem weiteren Schritt der Kalzinierung werden die Katalysator-Pellets in Form von Kugeln oder Extrudaten an Luft oder Sauerstoff bei Temperaturen von 620 bis 850 °C, besonders bevorzugt bei etwa 750 °C, über eine Dauer von 10 bis 24 Stunden kalziniert, um die gewünschte Kalaysatorzusammensetzung zu erhalten. Diese Folge von Wärmebehandlungsschritten wird bevorzugt, wenngleich der Katalysator auch erzeugt werden kann ohne den Kalzinierschritt bei 620 bis 850 °C vor der Formgebung. In diesem Fall wird der Katalysator hergestellt durch Imprägnieren eines geeigneten Trägermaterials. Entsprechend dem zuvor beschriebenen Verfahren wird das Kalzinieren zunächst im Temperaturbereich von 250 - 600°C und anschließend im Temperaturbereich von 620 - 850 °C ausgeführt.

Als Trägermaterial für den Katalysator kommen insbesondere Kieselerde und Tonerde in Frage sowie Siliziumkarbid, Siliziumnitrid und besonders bevorzugt eine der X- oder Y-Komponenten in oxidischer Form, die vorstehend erwähnt wurden, oder Mischungen dieser Stoffe mit oder ohne Bindemittel. Bei Benutzung eines Trägermaterials, welches weder X- noch Y-Komponenten enthält, macht das eigentliche Katalysatormaterial einen Gewichtsanteil von etwa 10 - 90 % des Katalysators aus, während der Rest aus dem Trägermaterial oder Bindemittel besteht. Es empfiehlt sich. Zirkon und Hafnium in die Lösung in Form der Oxide (Pulver oder Pellets) einzubringen. Diese Komponenten können auch in Form von Hydroxiden, die gemischt mit anderen oben erwähnten netten Bindern als Bindemittel dienen, in die Katalysatorzusammensetzung eingebracht werden. Vorzugsweise die Lanthaniden (Cer, Lanthan, Neodym, Dysprosium, Praesodym) Zinn, Antimon und/oder Blei werden in die Lösung in Form von unlöslichen pulverförmigen Oxiden eingeführt. Einige andere wasserlösliche Verbindungen von solchen Elementen, welche benutzt werden können, schließen korrespondierende Nitrate, Hologenide oder Oxalate ein. Die vorzugsweise verwendeten Alkali-Komponenten sind Li, Na und/oder K. Sie werden eingebracht in Form von wasserlöslichen Nitraten oder Halogeniden. Als Halogene werden bevorzugt die Elemente Cl, Br und/oder J, wobei diese in Form von wasserlöslichen Ammonium-Salzen zugegeben werden.

Der Katalysator wird vorzugsweise in der nachfolgend beschriebenen generellen Herstellweise erzeugt:

Die Z-Komponenten, die Nitrate oder Halogene sind, werden in Wasser gelöst und bilden eine erste klare Lösung. Die A-Komponenten, die Ammonium-Salze sind, werden in der ersten Lösung gelöst, so daß eine zweite klare Lösung gebildet ist. Die X-Komponenten, die Oxide sind, werden mit der zweiten Lösung zu einer ersten Suspension vermischt. Die Y-Komponenten, die Oxide sind, werden in die erste Suspension eingebracht, so daß eine zweite Suspension entsteht. Auf der anderen Seite werden solche Y-Verbindungen, die lösliche Nitrate oder Halogenide sind, teilweise in der zweiten Lösung gelöst. Nach Mischen und Erhitzen der zweiten Suspension für eine Dauer von ungefähr 15 Minuten bei einer Temperatur von ungefähr 80°C wird das Wasser verdampft, wobei dies vorzugsweise im Vacuum unter ständigem Rühren (in einem Rotationsverdampfer) erfolgt, so daß die Trocknung schnell erreicht wird. Das Trocknen könnte aber auch an Luft durchgeführt werden. Wenn der Katalysator auf einem Träger hergestellt wird, ist es zweckmäßig, die Imprägnierung mit separaten, klaren, gefilterten Lösungen der Y-, Z- und A-Komponenten-Verbindungen schrittweise mit zwischenzeitlicher Trocknung durchzuführen, um eine Ausfällung in der Imprägnierlösung zu vermeiden.

Die Größe und Aktivität der Oberfläche des Katalysators hängen ab von der Auslaugungszeit. d.h. von der Zeit, die benutzt wird, um die zweite Suspension bis zur Trockne einzudampfen. Zusammensetzungen, die über eine relativ lange Zeitperiode (z.B. 30 Minuten oder mehr) vor dem Trocknen bei 130°C auslaugen können, unterliegen im allgemeinen einem Kornwachstum mit einem Verlust an Oberflächengröße. Wenn der Katalysator in Form von Extrudaten genutzt werden soll, wird das verdampfte Material nach dem Trocknen bei z.B. 130°C und dem Kalzinieren bei z.B. 500°C vermischt mit Aluminium-, Titan- und/oder Zirkon-Hydroxid und/oder mit kolloidal verteilter Kieselerde (silica sol), durch Zugabe einer gewünchten Menge an Salpetersäure (oder im Falle eines Kieselerde-Bindemittels Ammoniak), plastifiziert durch Zugabe von Polyäthylenglykol, Cellulose oder anderen Ausbrennverbindungen und extrudiert durch eine Lochplatte mit anschließender Trocknung und Kalzinierung.

Der Katalysator gemäß der Erfindung sollte eine oder mehrere Metallkomponenten enthalten, die dicht unter ihrer hochstmöglichen Oxidationsstufe sind. Die Kalzinierung wird ausgeführt unter Durchleitung eines Sauerstoff enthaltenden Gases über die trockenen Feststoffe, die aus der Suspension gewonnen wurden, um die Reduktionstätigkeit des Reduktionsmittels wie etwa $NH_3$ oder organische Reduktionsmittel zu steuern, die in das System für die Plastifizierung und Formung zu Kügelchen eingebracht wurden. Die Durchflußrate des Gases kann experimentell für die Kalzinier-Vorrrichtung und die Mengen der benutzten Feststoffe bestimmt werden, um die Eigenschaften des zu erzeugenden Katalysators zu optimieren. Eine oder mehrere freie Valenzen der Metalle im Katalysator werden besetzt durch ein oder mehrere Oxide und Halogenide. Generell kann der Katalysator - mit oder ohne Trägermaterial oder extrudiert - in einem Festbett- oder einem Wirbelbettreaktor benützt werden.

Das Verfahren gemäß der Erfindung soll im folgenden naher beschrieben werden.

Als Einsatzmaterial können Ethan, Propan, Butan, Isobutan und Pentan sowie deren Mischungen in unterschiedlicher Zusammensetzung aus beliebigen Quellen verwendet werden. Der Gasstrom kann auch beträchtliche Mengen an Kohlenmonoxid, Stickstoff, Methan, Ethan, Äthylen und $C_3$-$C_5$-Alkenen mit jeweils mehr als 5 Volumenprozent sowie Wasser in Form von Dampf enthalten. Die eingesetzte Reaktionsmischung zur Ausführung des Verfahrens ist im allgemeinen wie folgt: 1 mol $C_2$-$C_5$-Paraffine, 0,05 - 2,0 mol molekularer Sauerstoff entweder als reiner Sauerstoff oder in Form von Luft oder einem anderen Sauerstoff enthaltenden Gas, 0 - 5 mol Stickstoff, 0,1 - 5 mol Verdünnungsmittel für die Reaktion, vorzugsweise Dampf, und 0 - 0,01 mol Halogene (Cl, Br oder I) in Form von organischen Halogeniden (Chloroform, Dichlorethan usw.). Dampf wird genutzt sowohl als Verdünnungsmittel für die Reaktion, als auch als Wärmemoderator für die Reaktion und als Reaktant zur Verbesserung der Olefinausbeute. Die Halogene werden benutzt als Stabilisierer, um einer Deaktivierung des Katalysators vorzubeugen. Bei hohen Reaktionstemperaturen können Halogene aus dem Feststoff ausgedampft werden. Das erfordert die Zugabe von Halogenen in das Einsatzmaterial. Auch andere Gase können als Reaktionsverdünnungsmittel oder Wärmemoderator benutzt werden, beispielsweise Helium, Kohlendioxid und Methan. Wasser bewirkt die Bildung einer gewissen Menge an Butyr-, Acryl- und Acetaldehyd aus den erzeugten Olefinen. Ihre Menge überschreitet nicht den Wert von 5 %.

Die gasförmige Reaktionsmischung wird vor der Einführung in die Reaktionszone gleichmäßig vermischt. Die Komponenten können vorgewärmt werden - einzeln oder nach der Mischung - bevor sie in die Reaktionszone, die eine Temperatur von 400°C bis etwa 700°C haben sollte, eingeführt werden. Die Reaktionszone kann einen Druck von etwa 1 - 5 bar, eine Temperatur von 400 - 700°C, vorzugsweise 520 - 650 °C und besonders bevorzugt von 580 - 640 °C aufweisen, während die gewichtsbezogene Raumgeschwindigkeit WHSV (berechnet auf der Basis des Paraffin-Flusses) im Bereich 0,1 - 20h$^{-1}$, vorzugsweise im Bereich 1 - 4h$^{-1}$ liegt. Allgemein gilt, daß der Prozeß ausgeführt werden kann in einer einzigen Stufe, wobei der gesamte Sauerstoff für die Rekation zusammen mit dem Verdünnungsmittel zugegeben wird. Es ist erwünscht, ohne ein inertes Verdünnungsmittel wie etwa Stickstoff zu arbeiten, damit die Abtrennung der erzeugten Olefine erleichtert wird. Wenn kein Verdünnungsmittel benutzt wird, können einige Probleme entstehen, weil die große Menge an Sauerstoff gefährliche Bedingungen schaffen kann, die eine Explosion und eine Verminderung der Selektivität des Prozesses für Olefine begünstigen. Eine Ausführung des Prozesses in mehreren Stufen erlaubt es, den benötigten Sauerstoff für die gesamte Reaktion des Paraffins in verschiedenen Stufen einzuführen und dadurch die Entstehung möglicherweise gefährlicher Bedingungen zu vermeiden.

Die Erfindung wird an den nachfolgenden Beispielen weiter erläutert. Die Versuche wurden ausgeführt in einem Röhrenreaktor unter Benutzung verschiedener Katalysatoren und den folgenden Bedingungen:

Die Zusammensetzung der zugeführten Gasmischung wies 20 Vol.-% LPG-Flüssiggas (50 Vol.-% Propan, 25 Vol.-% Butan, 25 Vol.-% Isobutan) oder einzelne $C_3$- und $C_4$-Paraffine, 20 Vol.-% Sauerstoff und 60 Vol.-% Wasser in Form von Dampf auf. Die gewichtsbezogene Raumgeschwindigkeit des Paraffins betrug ungefähr 6 h$^{-1}$ bei einem Gesamtdruck von 1 bar. Der Reaktor bestand aus einer Quartz-Röhre mit 15 mm Durchmesser, die in einem elektrischen Ofen erhitzt wurde, wobei die Temperatur in der isothermen Zone, in der der Katalysator zwischen zwei Lagen von Quartzpartikeln angeordnet war, etwa 590°C betrug. Der Reaktor enthielt 1 g des Katalysators, der mit 5 g Quartzpartikeln vermischt war. Das Wasser und Spuren von flüssigen Produkten wurden in einer Kondensationsvorrichtung bei - 20°C kondensiert.

Die gasförmigen Produkte wurden analysiert. In allen Fällen wurden die Prozentsätze der Kohlenwasserstoffkonversion (X) und der Olefin-Selektivität (S) wie folgt berechnet:

$$X = 100 - \Sigma\, C_{pi}$$

$$S = \frac{\Sigma C_{oi}}{X} * 100\%$$

wobei $C_{pi}$ und $C_{oi}$ die Gewichtsprozente der Paraffine und Olefine in der Kohlenwasserstoff-Mischung am Reaktorausgang sind.

**Beispiel 1**

Ein Katalysator mit folgender Zusammensetzung wurde hergestellt:

$$Zr_{0.62}Dy_{0.04}Li_{0.46}Cl_{0.24}O_x$$

Eine Menge von 4,23 g Lithiumnitrat wurde in 150 ml Wasser gegeben, auf 80°C erhitzt und 15 Minuten lang gerührt, um eine erste klare Lösung zu erhalten. Eine Menge von 2,3 g Ammoniumchlorid wurde in die erste Lösung bei 80°C gegeben und 20 Minuten lang gerührt, um eine zweite klare Lösung zu erhalten. Eine Menge von 1,0 g Zirkonoxid (Baddeleyit) wurde in die zweite Lösung gegeben und bei 80°C 10 Minuten lang gerührt, um eine erste Suspension zu erhalten. Eine Menge von 1,0 g Dysprosiumoxid wurde zu der ersten Suspension gegeben und 15 Minuten lang bei 80°C gerührt, um eine letzte Suspension zu erhalten. Das Wasser in der letzten Suspension wurde unter ständigem Rühren verdampft. Der feuchte Feststoff wurde getrocknet bei Raumtemperatur, gebrochen und auf eine Korngrößenfraktion von 400 - 800 µm abgesiebt sowie über Nacht in einem Ofen bei 130°C getrocknet. Das getrocknete Material wurde in einem Ofen bei einer Temperatur von 500°C zwei Stunden lang an fließender Luft kalziniert. Die Temperatur wurde danach für 16 Stunden auf 750°C angehoben. Das kalzinierte Material wurde in einem Kühlraum abgekühlt, zerkleinert und zu Pellets von 2,5 x 0,5 cm gepreßt. Die Pellets wurden zerkleinert, auf eine Partikelgröße von 400 - 800 µm abgesiebt und nochmals an Luft bei 750°C über 16 Stunden kalziniert. Der Katalysator wurde für die oxidative Dehydrierung einer Paraffinmischung gemäß der vorstehend beschriebenen Verfahrensweise getestet. Die Ergebnisse sind in Tabelle 2 dargestellt.

**Beispiel 2**

Unter Benutzung der im Beispiel 1 beschriebenen Vorgehensweise wurde ein Katalysator vorbereitet, der folgende Zusammensetzung aufwies:

$$Zr_{0.61}Dy_{0.08}Li_{0.43}Cl_{0.15}O_x$$

Es wurden wieder die gleichen Stoffmengen benutzt, außer daß 2,0 g Dysprosiumoxid eingesetzt wurde, so daß der hergestellte Katalysator einen höheren Dysprosiumgehalt als der Katalysator im Beispiel 1 hatte. Die Ergebnisse des Tests mit diesem Katalysator bei der oxidativen Dehydrierung einer LPG-Mischung sind in Tabelle 2 wiedergegeben.

**Beispiel 3**

Unter Benutzung der Verfahrensweise des Beispiels 1 wurde ein Katalysator mit folgender Zusammensetzung hergestellt:

$$Zr_{0.62}Dy_{0.04}Li_{0.86}Cl_{0.28}O_x$$

Es wurden dieselben Stoffmengen benutzt, außer daß 8,46 g Lithiumnitrat eingesetzt wurde, so daß der hergestellte Katalysator einen höheren Lithiumgehalt als der Katalysator von Beispiel 1 hatte. Die Ergebnisse des Tests dieses Katalysators bei der oxidativen Dehydrierung einer LPG-Mischung sind in Tabelle 2 wiedergegeben.

**Beispiele 4 - 10**

Die Beispiele 4 - 10 wurden ausgeführt unter Benutzung derselben Vorgehensweise, wie sie im Beispiel 1 angewandt wurde, mit denselben Mengen an X-, Y-, Z- und A-Komponenten in g-atom, wobei die Elemente der X-, Y-, Z- und A-Komponenten variierten. Tabelle 1 zeigt die X-, Y-, Z- und A-Komponenten, die X-, Y-, Z- und ASalze oder Oxide, die Gewichte der X-, Y-, Z- und A-Salze oder Oxide und die Zusammensetzung der Katalysatoren für die Beispiele 4 - 10. Die Ergebnisse der Tests mit diesen Katalysatoren sind in Tabelle 2 wiedergegeben.

**Beispiel 11**

Ein Katalysator, der gemäß der Verfahrensweise aus Beispiel 1 hergestellt wurde, wurde bei der oxidativen Dehydrierung von Propan unter den vorstehend beschriebenen Bedingungen getestet. Die Konversion von Propan betrug 79 %, die Selektivität für $C_2$-$C_3$-Olefine 61 %.

**Beispiel 12**

Ein Katalysator, der gemäß dem Verfahren von Beispiel 1 hergestellt wurde, wurde bei der oxidativen Dehydrierung von Isobutan unter den vorstehend beschriebenen Bedingungen getestet. Die Konversion von Isobutan betrug 77 %, die Selektivität für $C_2$-$C_4$-Olefine 59%.

**Beispiel 13**

Ein Katalysator der folgenden Zusammensetzung wurde durch Aufbringung eines Teils der aktiven Oxide auf einem extrudierten Trägermaterial (2,5 mm Durchmesser) hergestellt, wobei das Trägermaterial $ZrO_2$ und $Al_2O_3$ (letzteres als Bindemittel benutzt) umfaßte:

$$Zr_{0.62}Dy_{0.012}Li_{0.14}Cl_{0.18}O_x\text{-}Al_2O_3$$

13 g Zr-Hydroxid-Pellets, die mit Al-Hydroxid koextrudiert waren (Produkt 706/03 von MEL Chemicals), wurden bei 550°C an Luft über 5 Stunden kalziniert, um beide Hydroxide zu dehydratisieren. Nach der Kalzinierung enthielt das Trägermaterial 85 Gew.-% $ZrO_2$ und 15 Gew.-% $Al_2O_3$.

15,5g $Dy(No_3)_3$ x 5 $H_2O$ und 27,5g $LiNO_3$ wurden in 30 g Wasser bei Raumtemperatur gelöst. Die kalzinierten Pellets des Zr-Al-Trägers wurden bei 50 mm Hg evakuiert für eine Dauer von 0,5 Stunden, und dann wurde die Dy-Li-Lösung zugegeben. Nach einer Kontaktzeit von 1 Stunde wurden die Pellets von der Lösung getrennt, bei 110°C über eine Zeit von 12 Stunden getrocknet und dann an Luft bei 550°C 3 Stunden lang kalziniert.

4,8 $NH_4Cl$ wurden bei Raumtemperatur in 15 $cm^3$ Wasser gelöst. Diese Lösung wurde, wie zuvor beschrieben, für die zweite Imprägnierung des Dy-Li-Zr-Al-Trägers benutzt. Nach Trocknung bei 110°C und Kalzinierung bei 550°C wurden die Pellets nochmals bei 750°C für eine Zeit von 15 Stunden kalziniert.

Alle Beispiele zeigen die hohe Wirksamkeit des neuen Katalysators, die sich in hohen Konversionsraten der paraffinischen Kohlenwasserstoffe und in einer hohen Selektivität für Olefine äußert. Die Ausbeute an Olefinen ist bemerkenswert hoch und beläuft sich auf Werte oberhalb von 40% (verglichen mit 20 - 30% in konventionellen Prozessen). Darüber hinaus wird das erfindungsgemäße Verfahren bei vergleichsweise niedrigen Temperaturen und unter nahezu isothermen Bedingungen ausgeführt. Das wirkt sich in einer bedeutsamen Verminderung des Energieverbrauchs aus.

Tabelle 1

| Beispiel | X | | Y | | Z | | A | | Katalysatorzusammensetzung | | | | | | | |
|---|---|---|---|---|---|---|---|---|---|---|---|---|---|---|---|---|
| | Salz | Gew./g | Salz | Gew./g | Salz | Gew./g | Salz | Gew./g | | | | | | | | |
| 4 | $TiO_2$ | 6.5 | $Dy_2O_3$ | 0.75 | $LiNO_3$ | 3.2 | $NH_4Cl$ | 2.3 | Ti | 0.55 | Dy | 0.03 | Li | 0.35 | Cl | 0.21 |
| 5 | $ZrO_2$ | 10.0 | $La_2O_3$ | 0.22 | $NaNO_3$ | 2.66 | $NH_4Br$ | 3.7 | Zr | 0.60 | La | 0.01 | Na | 0.24 | Br | 0.21 |
| 6 | $ZrO_2$ | 10.0 | $Nd_2O_3$ | 0.23 | LiCl | 1.22 | $NH_4Cl$ | 2.3 | Zr | 0.60 | Nd | 0.01 | 2Li | 0.22 | Cl | 0.45 |
| 7 | $TiO_2$ | 7.1 | $Pr(NO_3)_3$ | 0.90 | KCl | 9.70 | $NH_4Cl$ | 2.3 | Ti | 0.60 | Pr | 0.02 | K | 1.00 | Cl | 0.50 |
| 8 | $ZrO_2$ | 10.0 | $Dy(NO_3)_3$ | 0.47 | $LiNO_3$ | 2.30 | $NH_4I$ | 8.8 | Zr | 0.60 | Dy | 0.02 | Li | 0.25 | I | 0.34 |
| 9 | $TiO_2$ | 7.1 | Sn(II)-oxalate | 6.56 | $NaNO_3$ | 3.32 | $NH_4Cl$ | 2.3 | Ti | 0.60 | Sn | 0.20 | Na | 0.30 | Cl | 0.26 |
| 10 | $ZrO_2$ | 10.0 | Sb(II)-oxalate | 5.00 | $LiNO_3$ | 3.90 | $NH_4Br$ | 6.7 | Zr | 0.60 | Sb | 0.15 | Li | 0.42 | Br | 0.38 |

Tabelle 2

| Beispiel | Metalle | | | Halogen | WHSV (LPG) ($h^{-1}$) | LPG Konversion (%) | Selektivität für $C_2$- $C_4$ Olefine (%) |
|---|---|---|---|---|---|---|---|
| | X | Y | Z | | | | |
| 1 | Zr | Dy | Li | Cl | 6 | 75 | 62 |
| 2 | Zr | Dy | Li | Cl | 6 | 78 | 60 |
| 3 | Zr | Dy | Li | Cl | 4 | 62 | 74 |
| 4 | Ti | Dy | Li | Cl | 6 | 81 | 64 |
| 5 | Zr | La | Na | Br | 6 | 81 | 64 |
| 6 | Zr | Nd | Li | Cl | 6 | 65 | 71 |
| 7 | Ti | Pr | K | Cl | 6 | 59 | 82 |
| 8 | Zr | Dy | Li | I | 12 | 75 | 76 |
| 9 | Ti | Sn | Na | Cl | 6 | 49 | 53 |
| 10 | Zr | Sb | Li | Br | 6 | 46 | 68 |
| 13 | Zr | Dy | Li | Cl | 2 | 63 | 65 |

**Patentansprüche**

1. Kalzinierter oxidischer Katalysator zum oxidativen Dehydrieren/Cracken von paraffinischen Kohlenwasserstoffen, die 2 bis 5 Kohlenstoffatome enthalten, zu Olefinen, wobei die aktive Komponente des Katalysators eine Zusammensetzung gemäß der folgenden Formel aufweist:

$$X_a Y_b Z_c A_d O_x,$$

wobei

X wenigstens ein Metall aus der Gruppe Zirkonium und Hafnium ist,
Y wenigstens ein Metall aus den Gruppen der Lanthaniden und IVa und Va ist,
Z wenigstens ein Metall aus der Gruppe I ist,
A wenigstens ein Halogen aus der Gruppe VII des periodischen Systems und O Sauerstoff ist
und wobei

a eine Zahl im Bereich von 0,4 - 0,9,
b eine Zahl im Bereich von 0,005 - 0,3,
c eine Zahl im Bereich von 0,05 - 1,5,
d eine Zahl im Bereich von 0,05 - 0,8 und
x eine Zahl ist, die sich nach den Valenz-Erfordernissen der Metalle
X, Y und Z sowie der Menge der Halogene richtet.

2. Katalysator nach Anspruch 1,
dadurch gekennzeichnet,
daß

a eine Zahl im Bereich 0,5 - 0,8,
b eine Zahl im Bereich 0,01 - 0,2,
c eine Zahl im Bereich 0,1 - 1,0 und
d eine Zahl im Bereich 0,1 - 0,5 ist.

3. Katalysator nach Anspruch 1 oder 2,
dadurch gekennzeichnet,

daß der Katalysator in folgenden Schritten hergestellt wird:

a) Auflösen der löslichen Bestandteile und Dispergieren der unlöslichen Bestandteile, so daß das gewünschte mol-Verhältnis der Elemente im Katalysator erreicht wird,

b) Abtrennung des Lösungsmittels von der Lösung,

c) Trocknen des nassen Katalysators vorzugsweise bei 110 - 150°C und besonders bevorzugt bei 130°C, über eine Zeit von 10 bis 24 Stunden, vorzugsweise an Luft oder Sauerstoff,

d) Zerkleinerung des erhaltenen trockenen Feststoffs aus Schritt c) zu einem Pulver, vorzugsweise mit einer Korngröße im Bereich von 400 bis 800 μm,

e) Kalzinieren des in Schritt d) erhaltenen Pulvers in einer Sauerstoff enthaltenden Atmosphäre bei einer Temperatur im Bereich von 250 bis 600°C, vorzugsweise bei 500°C, für eine Zeit von 0,5 bis 5 h,

f) Formung von Katalysatorpellets aus dem Pulver von Schritt e) durch Pressen und Einformung zu Kügelchen unter Zusatz von Feuchtigkeit, oberflächenaktiven Stoffen und Plastifizierern oder durch Extrusion unter Zugabe eines Bindemittels,

g) Kalzinieren der gebildeten Pellets, vorzugsweise an Luft oder Sauerstoff, bei einer Temperatur im Bereich von 600 bis 800°C, vorzugsweise bei 750°C, über eine Zeit von 10 bis 24 Stunden.

4. Katalysator nach Anspruch 3,
dadurch gekennzeichnet,
daß das kalzinierte Pulver aus Schritt e) vor der Formung zusätzlich bei einer Temperatur im Bereich von 620 - 850°C, vorzugsweise bei 750°C, über eine Dauer von 10 - 24 Stunden kalziniert wird.

5. Katalysator nach Anspruch 1 oder 2,
dadurch gekennzeichnet,
daß der Katalysator aufgebracht auf ein Trägermaterial in folgenden Schritten hergestellt wird:

a) Auflösen der löslichen Bestandteile des Katalysators und Imprägnieren des Trägermaterials mit der Lösung, so daß das gewünschte mol-Verhältnis der Elemente im Katalysator erreicht wird,

b) Abtrennung des Lösungsmittels von der Lösung,

c) Trocknen des nassen Katalysators, vorzugsweise bei 110 - 150°C und besonders bevorzugt bei 130°C, über eine Zeit von 10 - 24 Stunden, vorzugsweise an Luft oder Sauerstoff,

d) Kalzinieren des Katalysators aus Schritt c), vorzugsweise an Luft oder Sauerstoff bei einer Temperatur im Bereich von 250 - 600°C, vorzugsweise bei 500°C, über eine Zeit von 0,5 - 5 Stunden,

e) weiteres Kalzinieren des Katalysators aus Schritt d), vorzugsweise an Luft oder Sauerstoff, bei einer Temperatur im Bereich von 620 - 850°C, vorzugsweise bei 750°C, für eine Dauer von 10 - 24 Stunden.

6. Katalysator nach Anspruch 5,
dadurch gekennzeichnet,
daß wenigstens eine der löslichen Komponenten, vorzugsweise die Halogen-Komponente (A), in separaten Schritten a) - d) auf das Trägermaterial aufgebracht wird, bevor der abschließende Kalzinierschritt e) ausgeführt wird.

7. Katalysator nach Anspruch 5 oder 6,
dadurch gekennzeichnet,
daß als Trägermaterial eines oder eine Mischung der Verbindungen aus der Gruppe Kieselerde, Tonerde, Siliziumkarbid und Siliziumnitrid und/oder vorzugsweise aus den Elementen der X- und Y-Komponente, die mit Kieselerde, Tonerde oder Siliziumkarbid als Bindemittel vermischt wird, benutzt wird.

8. Katalysator nach Anspruch 6 oder 7,

dadurch gekennzeichnet,
daß die aktive Komponente des Katalysators 10 bis 90 Gew.-% des Katalysators ausmacht (Rest Trägermaterial).

9. Katalysator nach Anspruch 3 oder 4,
dadurch gekennzeichnet,
daß die aktive Komponente des Katalysators 10 - 90 Gew.-% des Katalysators ausmacht (Rest Bindemittel).

10. Katalysator nach einem der Ansprüche 1 - 9,
dadurch gekennzeichnet,
daß Y wenigstens eines der Metalle aus der Gruppe Cer, Lanthan, Neodym, Dysprosium, Zinn, Praseodym, Antimon und Blei ist.

11. Katalysator nach einem der Ansprüche 1 - 10,
dadurch gekennzeichnet,
daß Z wenigstens eines der Metalle aus der Gruppe Lithium, Natrium und Kalium ist.

12. Katalysator nach einem der Ansprüche 1 - 11,
dadurch gekennzeichnet,
daß A wenigstens ein Halogen aus der Gruppe Chlor, Brom und Jod ist.

13. Verwendung eines Katalysators nach Anspruch 1 in einem Verfahren zum oxidativen Dehydrieren/Cracken von paraffinischen Kohlenwasserstoffen, die zwei bis fünf Kohlenstoffatome ($C_2$ - $C_5$) enthalten, zu Olefinen durch Kontaktieren der Kohlenwasserstoffe mit einem molekularen Sauerstoff enthaltenden Gas und einem Verdünnungsmittel, insbesondere Wasserdampf, in einer Reaktionszone über dem Katalysator mit der Maßgabe, daß die Temperatur im Bereich von 400 bis 700°C, der Druck im Bereich von 1 bis 5 bar, die gewichtsbezogene Raumgeschwindigkeit (WHSV) der paraffinischen Kohlenwasserstoffe im Bereich von 0,1 bis 20 $h^{-1}$, das mol-Verhältnis von Sauerstoff zu den Kohlenwasserstoffen im Bereich von 0,01 bis 5 und das mol-Verhältnis des Verdünnungsmittels zu den Kohlenwasserstoffen im Bereich von 0,1 bis 5 gehalten werden.

14. Verwendung nach Anspruch 13,
dadurch gekennzeichnet,
daß die Temperatur im Bereich von 500 bis 600°C, insbesondere im Bereich von 540 bis 590°C, gehalten wird.

15. Verwendung nach Anspruch 13 oder 14,
dadurch gekennzeichnet,
daß die WHSV im Bereich von 5 bis 7 $h^{-1}$ gehalten wird.

16. Verwendung nach einem der Ansprüche 13 - 15,
dadurch gekennzeichnet,
daß der Druck im Bereich von 1 bis 2 bar gehalten wird.

17. Verwendung nach einem der Ansprüche 13 - 16,
dadurch gekennzeichnet,
daß das Sauerstoff enthaltende Gas reiner Sauerstoff ist.

18. Verwendung nach einem der Ansprüche 13 - 17,
dadurch gekennzeichnet,
daß das Sauerstoff enthaltende Gas in wenigstens zwei Schritten zugegeben wird.

19. Verwendung nach einem der Ansprüche 13 - 18,
dadurch gekennzeichnet,
daß die paraffinischen Kohlenwasserstoffe LPG (eine Mischung von Propan, n-Butan und Isobutan) sind.

20. Verwendung nach einem der Ansprüche 13 - 19,
dadurch gekennzeichnet,
daß zusätzlich organische Halogenide in der Reaktionszone anwesend sind.

21. Verwendung nach einem der Ansprüche 13 - 19,

dadurch gekennzeichnet,
daß zusätzlich Stickstoff in der Reaktionszone anwesend ist.

22. Verwendung nach einem der Ansprüche 13 - 21,
dadurch gekennzeichnet,
daß das mol-Verhältnis von Wasserdampf zu den Kohlenwasserstoffen im Bereich von 0,1 bis 4 gehalten wird.

23. Verwendung nach einem der Ansprüche 13 - 22,
dadurch gekennzeichnet,
daß das mol-Verhältnis von Sauerstoff zu den Kohlenwasserstoffen im Bereich von 0,01 bis 1,0 gehalten wird.

24. Verwendung nach einem der Ansprüche 13 - 20,
dadurch gekennzeichnet,
daß das mol-Verhältnis der Halogene (Cl, Br oder J) zu den Kohlenwasserstoffen im Bereich von 0 bis 0,01 gehalten wird.

**Claims**

1. A calcined oxidic catalyst for the oxidative dehydrogenation/cracking of paraffinic hydrocarbons which contain 2 to 5 carbon atoms to produce olefins, the active component of the catalyst having a composition in accordance with the following formula:

$$X_a Y_b Z_c A_d O_x,$$

wherein

X is at least one metal of the group consisting of zirconium and hafnium,
Y is at least one metal of the groups consisting of the lanthanides and IVa and Va,
Z is at least one metal of group I,
A is at least one halogen of group VII of the periodic system and O is oxygen,
and wherein

a is a number in the range from 0.4 - 0.9,
b is a number in the range from 0.005 - 0.3,
c is a number in the range from 0.05 - 1.5,
d is a number in the range from 0.05 - 0.8, and
x is a number which depends on the valency requirements of the metals X, Y and Z and the quantity of halogens.

2. A catalyst according to Claim 1, characterised in that

a is a number in the range 0.5 - 0.8,
b is a number in the range 0.01 - 0.2,
c is a number in the range 0.1 - 1.0, and
d is a number in the range 0.1 - 0.5.

3. A catalyst according to Claim 1 or 2, characterised in that the catalyst is produced in the following steps:

a) dissolving of the soluble constituents and dispersing the insoluble constituents, so that the desired molar ratio of the elements is obtained in the catalyst,

b) separation of the solvent from the solution,

c) drying of the wet catalyst preferably at 110 - 150°C and particularly preferably at 130°C, over a period of 10 to 24 hours, preferably in air or oxygen,

d) comminution of the resulting dry solid from step c) to form a powder, preferably having a grain size in the range from 400 to 800 µm,

e) calcining the powder obtained in step d) in an oxygen-containing atmosphere at a temperature in the range from 250 to 600°C, preferably at 500°C, for a period of 0.5 to 5 hours,

f) forming catalyst pellets from the powder of step e) by pressing and shaping into small spheres with the addition of moisture, surface-active substances and plasticisers, or by extrusion with the addition of a binder,

g) calcining of the resulting pellets, preferably in air or oxygen, at a temperature in the range from 600 to 800°C, preferably at 750°C, over a period of 10 to 24 hours.

4.  A catalyst according to Claim 3, characterised in that the calcined powder of step e) is additionally calcined at a temperature in the range from 620 - 850°C, preferably at 750°C, for a period of 10 - 24 hours, before forming.

5.  A catalyst according to Claim 1 or 2, characterised in that the catalyst is produced, applied to a support material, in the following steps:

a) dissolving of the soluble constituents of the catalyst and impregnation of the support material with the solution, so that the desired molar ratio of the elements is obtained in the catalyst,

b) separation of the solvent from the solution,

c) drying of the wet catalyst, preferably at 110 - 150°C and particularly preferably at 130°C, over a period of 10 - 24 hours, preferably in air or oxygen,

d) calcining the catalyst of step c), preferably in air or oxygen, at a temperature in the range from 250 - 600°C, preferably at 500°C, for a period of 0.5 - 5 hours,

e) further calcining of the catalyst of step d), preferably in air or oxygen, at a temperature in the range from 620 - 850°C, preferably at 750°C, over a period of 10 - 24 hours.

6.  A catalyst according to Claim 5, characterised in that at least one of the soluble components, preferably the halogen component (A), is applied to the support material in separate steps a) - d) before the final calcining step e) is performed.

7.  A catalyst according to Claim 5 or 6, characterised in that one compound or a mixture of the compounds from the group consisting of silica, alumina, silicon carbide and silicon nitride and/or preferably of the elements of the X and Y components which are mixed with silica, alumina or silicon carbide as binder is used as the support material.

8.  A catalyst according to Claim 6 or 7, characterised in that the active component of the catalyst makes up 10 to 90% by weight of the catalyst (remainder support material).

9.  A catalyst according to Claim 3 or 4, characterised in that the active component of the catalyst makes up 10 - 90% by weight of the catalyst (remainder binder).

10. A catalyst according to one of Claims 1 - 9, characterised in that Y is at least one of the metals from the group consisting of cerium, lanthanum, neodymium, dysprosium, tin, praseodymium, antimony and lead.

11. A catalyst according to one of Claims 1 - 10, characterised in that Z is at least one of the metals from the group consisting of lithium, sodium and potassium.

12. A catalyst according to one of Claims 1 - 11, characterised in that A is at least one halogen from the group consisting of chlorine, bromine and iodine.

13. The use of a catalyst according to Claim 1 in a method for the oxidative dehydrogenation/cracking of paraffinic hydrocarbons which contain two to five carbon atoms ($C_2$-$C_5$) to produce olefins, by contacting the hydrocarbons with a gas containing molecular oxygen and a diluent, in particular water vapour, in a reaction zone over the catalyst with the proviso that the temperature is kept within the range from 400 to 700°C, the pressure within the range from 1 to 5 bar, the weight-related space velocity (WHSV) of the paraffinic hydrocarbons within the range from 0.1 to 20 $h^{-1}$, the molar ratio of oxygen to the hydrocarbons within the range from 0.01 to 5 and the molar ratio of the diluent to the hydrocarbons within the range from 0.1 to 5.

14. The use according to Claim 13, characterised in that the temperature is kept within the range from 500 to 600°C, in particular in the range from 540 to 590°C.

15. The use according to Claim 13 or 14, characterised in that the WHSV is kept within the range from 5 to 7 h$^{-1}$.

16. The use according to one of Claims 13 - 15, characterised in that the pressure is kept within the range from 1 to 2 bar.

17. The use according to one of Claims 13 - 16, characterised in that the oxygen-containing gas is pure oxygen.

18. The use according to one of Claims 13 - 17, characterised in that the oxygen-containing gas is added in at least two steps.

19. The use according to one of Claims 13 - 18, characterised in that the paraffinic hydrocarbons are LPG (a mixture of propane, n-butane and isobutane).

20. The use according to one of Claims 13 - 19, characterised in that additionally organic halides are present in the reaction zone.

21. The use according to one of Claims 13 - 19, characterised in that additionally nitrogen is present in the reaction zone.

22. The use according to one of Claims 13 - 21, characterised in that the molar ratio of water vapour to the hydrocarbons is kept within the range from 0.1 to 4.

23. The use according to one of Claims 13 - 22, characterised in that the molar ratio of oxygen to the hydrocarbons is kept within the range from 0.01 to 1.0.

24. The use according to one of Claims 13 - 20, characterised in that the molar ratio of the halogens (Cl, Br or I) to the hydrocarbons is kept within the range from 0 to 0.01.

**Revendications**

1. Catalyseur calciné agissant par voie d'oxydation pour déshydrogéner/craquer de façon oxydante des hydrocarbures paraffiniques, qui contiennent de 2 à 5 atomes de carbone, en oléfines, les composants actifs du catalyseur présentant une composition selon la formule suivante :

$$X_a Y_b Z_c A_d O_x$$

X étant au moins un métal du groupe zirconium et hafnium,
Y étant au moins un métal des groupes des lanthanides et IVa et Va,
Z étant au moins un métal du groupe I,
A étant au moins un halogène du groupe VII du système périodique et O étant de l'oxygène, et

a étant un nombre dans la plage de 0,4-0,9,
b étant un nombre dans la plage de 0,005-0,3,
c étant un nombre dans la plage de 0,05-1,5,
d étant un nombre dans la plage de 0,05-0,8, et
x étant un nombre qui est déterminé par les contraintes de valence des métaux X, Y et Z, ainsi que par la quantité des halogènes.

2. Catalyseur selon la revendication 1, caractérisé en ce que :

a est un nombre dans la plage de 0,5-0,8,
b est un nombre dans la plage de 0,01-0,2,

c est un nombre dans la plage de 0,1-1,0, et

d est un nombre dans la plage de 0,1-0,5.

3. Catalyseur selon la revendication 1 ou 2,
caractérisé en ce que le catalyseur est fabriqué selon les étapes suivantes :

a) dissolution des constituants solubles et dispersion des constituants insolubles, de sorte que le rapport molaire souhaité des éléments dans le catalyseur soit atteint,
b) séparation du solvant de la solution,
c) séchage du catalyseur humide avantageusement à 110-150°C et de façon plus particulièrement préférée à 130°C, pendant une durée de 10 à 24 heures, avantageusement sur de l'air ou de l'oxygène,
d) fragmentation de la matière solide sèche obtenue à partir de l'étape c) en une poudre, avantageusement ayant une grosseur de grains dans la plage de 400 à 800 μm,
e) calcination de la poudre obtenue dans l'étape d) dans une atmosphère contenant de l'oxygène à une température dans la plage de 250 à 600°C, avantageusement à 500°C, pendant une durée de 0,5 à 5 heures,
f) formation de granulés de catalyseur à partir de la poudre de l'étape e) par pressage et conformation en petites billes en ajoutant de l'humidité, des agents tensio-actifs et des plastifiants, ou par extrusion en ajoutant un liant,
g) calcination des granulés formés, avantageusement sur de l'air ou de l'oxygène, à une température dans la plage de 600 à 800°C, avantageusement à 750°C, pendant une durée de 10 à 24 heures.

4. Catalyseur selon la revendication 3,
caractérisé en ce que la poudre calcinée à partir de l'étape e) est calcinée de plus avant la déformation à une température dans la plage de 620-850°C, avantageusement à 750°C, pendant une durée de 10-24 heures.

5. Catalyseur selon la revendication 1 ou 2,
caractérisé en ce que le catalyseur, appliqué sur un matériau de support, est fabriqué selon les étapes suivantes :

a) dissolution des constituants solubles du catalyseur et imprégnation du matériau de support avec la solution, de sorte que le rapport molaire souhaité des éléments dans le catalyseur soit atteint,
b) séparation du solvant de la solution,
c) séchage du catalyseur humide avantageusement à 110-150°C et de façon plus particulièrement préférée à 130°C, pendant une durée de 10-24 heures, avantageusement sur de l'air ou de l'oxygène,
d) calcination du catalyseur de l'étape c), avantageusement sur de l'air ou de l'oxygène, à une température dans la plage de 250-600°C, avantageusement à 500°C, pendant une durée de 0,5-5 heures,
e) calcination ultérieure du catalyseur à partir de l'étape d), avantageusement sur de l'air ou de l'oxygène, à une température dans la plage de 620-850°C, avantageusement à 750°C, pendant une durée de 10-24 heures.

6. Catalyseur selon la revendication 5,
caractérisé en ce qu'au moins un des composants solubles, avantageusement le composant halogéné (A), est appliqué sur le matériau de support dans les étapes séparées a)-d), avant que l'étape finale de calcination e) soit effectuée.

7. Catalyseur selon la revendication 5 ou 6,
caractérisé en ce que, comme matériau de support, il est utilisé un ou un mélange des composés du groupe : silice, alumine, carbure de silicium et nitrure de silicium et/ou, avantageusement, des éléments du composant X et du composant Y, qui est mélangé avec de la silice, de l'alumine ou du carbure de silicium comme liant.

8. Catalyseur selon la revendication 6 ou 7,
caractérisé en ce que le composant actif du catalyseur constitue 10 à 90% en poids du catalyseur (le restant étant le matériau de support).

9. Catalyseur selon la revendication 3 ou 4,
caractérisé en ce que le composant actif du catalyseur constitue 10-90% en poids du catalyseur (le restant étant le liant).

10. Catalyseur selon une des revendications 1-9,

caractérisé en ce que Y est au moins un des métaux du groupe cérium, lanthane, néodyme, dysprosium, étain, praséodyme, antimoine et plomb.

11. Catalyseur selon une des revendications 1-10, caractérisé en ce que Z est au moins un des métaux du groupe lithium, sodium et potassium.

12. Catalyseur selon une des revendications 1-11, caractérisé en ce que A est au moins un halogène du groupe chlore, brome et iode.

13. Utilisation d'un catalyseur selon la revendication 1 dans un procédé pour déshydrogéner/craquer de façon oxydante des hydrocarbures paraffiniques, qui contiennent de 2 à 5 atomes de carbone ($C_2$ - $C_5$), en oléfines par mise en contact des hydrocarbures avec un gaz contenant de l'oxygène moléculaire et un agent de dilution, en particulier de la vapeur d'eau, dans une zone réactionnelle sur le catalyseur, sous réserve que la température soit maintenue dans la plage de 400 à 700°C, la pression dans la plage de 1 à 5 bars, la vitesse spatiale liée au poids des hydrocarbures paraffiniques dans la plage de 0,1 à 20 $h^{-1}$, le rapport molaire de l'oxygène aux hydrocarbures dans la plage de 0,01 à 5, et le rapport molaire de l'agent de dilution aux hydrocarbures dans la plage de 0,1 à 5.

14. Utilisation selon la revendication 13, caractérisée en ce que la température est maintenue dans la plage de 500 à 600°C, en particulier dans la plage de 540 à 590°C.

15. Utilisation selon la revendication 13 ou 14, caractérisée en ce que la vitesse spatiale liée au poids est maintenue dans la plage de 5 à 7 $h^{-1}$.

16. Utilisation selon une des revendications 13-15, caractérisée en ce que la pression est maintenue dans la plage de 1 à 2 bars.

17. Utilisation selon une des revendications 13-16, caractérisée en ce que le gaz contenant de l'oxygène est de l'oxygène pur.

18. Utilisation selon une des revendications 13-17, caractérisée en ce que le gaz contenant de l'oxygène est ajouté en au moins deux étapes.

19. Utilisation selon une des revendications 13-18, caractérisée en ce que les hydrocarbures paraffiniques sont du GPL (un mélange de propane, de n-butane et d'isobutane).

20. Utilisation selon une des revendications 13-19, caractérisée en ce que des halogénures organiques sont de plus présents dans la zone réactionnelle.

21. Utilisation selon une des revendications 13-19, caractérisée en ce que de l'azote est de plus présent dans la zone réactionnelle.

22. Utilisation selon une des revendications 13-21, caractérisée en ce que le rapport molaire de la vapeur d'eau aux hydrocarbures est maintenu dans la plage de 0,1 à 4.

23. Utilisation selon une des revendications 13-22, caractérisée en ce que le rapport molaire de l'oxygène aux hydrocarbures est maintenu dans la plage de 0,01 à 1,0.

24. Utilisation selon une des revendications 13-20, caractérisée en ce que le rapport molaire des halogènes (Cl, Br ou I) aux hydrocarbures est maintenu dans la plage de 0 à 0,01.